Europäisches Patentamt

European Patent Office

Office européen des brevets

(11) Veröffentlichungsnummer: **0 402 733**

**A2**

(12) **EUROPÄISCHE PATENTANMELDUNG**

(21) Anmeldenummer: **90110573.4**

(51) Int. Cl.⁵: **A61K 31/445**

(22) Anmeldetag: **05.06.90**

(30) Priorität: **10.06.89 DE 3919075**

(43) Veröffentlichungstag der Anmeldung:
**19.12.90 Patentblatt 90/51**

(84) Benannte Vertragsstaaten:
**AT BE CH DE DK ES FR GB GR IT LI LU NL SE**

(71) Anmelder: **Dr. Karl Thomae GmbH**
**Postfach 1755**
**D-7950 Biberach 1(DE)**

(72) Erfinder: **Engel, Wolfhard, Dr. Dipl.-Chem.**
**Mozartstrasse 13**
**D-7950 Biberach 1(DE)**
Erfinder: **Mayer, Norbert, Dr.**
**Friedrich-Ebert-Strasse 66**
**D-7950 Biberach 1(DE)**
Erfinder: **Doods, Henri, Dr.**
**Hornsteinweg 7**
**D-7951 Warthausen(DE)**
Erfinder: **Eberlein, Wolfgang, Dr. Dipl.-Chem.**
**Obere Au 6**
**D-7950 Biberach 1(DE)**
Erfinder: **Mihm, Gerhard, Dr. Dipl.-Chem.**
**Nickeleshalde 5/1**
**D-7950 Biberach 1(DE)**
Erfinder: **Rudolf, Klaus, Dr.**
**Marktplatz 38**
**D-7950 Biberach 1(DE)**

(54) **Mittel zur Behandlung von Erkrankungen des Zentralnervensystems und zur Förderung der cerebralen Durchblutung.**

(57) (±)-1-[4-[Ethyl[2-(4-methoxyphenyl)-1-methylethyl]amino]-1-oxobutyl]-N,N-dimethyl-4-piperidincarboxamid und (+)-1-[4-[Ethyl[2-(4-methoxyphenyl)-1-methylethyl]amino]-1-oxobutyl]-N,N-dimethyl-4-piperidincarboxamid, des weiteren die physiologisch verträglichen Säureadditionssalze dieser Verbindungen eignen sich zur Behandlung von Erkrankungen des Zentralnervensystems (e. g. des Morbus Alzheimer und Morbus Parkinson). Die Verbindungen zeigen auch antiemetische Eigenschaften und eignen sich angesichts günstiger Effekte auf die cerebrale Durchblutung zur Anwendung in der Geriatrie und bei Migräne.

EP 0 402 733 A2

## Mittel zur Behandlung von Erkrankungen des Zentralnervensystems und zur Förderung der cerebralen Durchblutung

Die Erfindung betrifft ein Mittel und dessen Verwendung zur Behandlung von Erkrankungen des Zentralnervensystems und zur Förderung der cerebralen Durchblutung, welches die Verbindungen (±)-1-[4-[Ethyl[2-(4-methoxyphenyl)-1-methylethyl]amino]-1-oxobutyl]-N,N-dimethyl-4-piperidincarboxamid (I) und (+)-1-[4-[Ethyl[2-(4-methoxyphenyl)-1-methylethyl]amino]-1-oxobutyl]-N,N-dimethyl-4-piperidincarboxamid (II) sowie deren physiologisch verträgliche Salze mit anorganischen oder organischen Säuren enthält.

In der europäischen Patentanmeldung Nr. 85 201 394.5 (Veröffentlichungs-Nr. 0 177 078) werden Verbindungen beschrieben, die als muscarinische Rezeptorantagonisten spezifische spasmolytische Eigenschaften besitzen und damit vorteilhaft zur Behandlung von Spasmen im Gastrointestinaltrakt eingesetzt werden können.

Es wurde überraschend gefunden, daß die in dem oben angeführten Patent enthaltenen Verbindungen

I. (±)-1-[4-[Ethyl[2-(4-methoxyphenyl)-1-methylethyl]amino]-1-oxobutyl]-N,N-dimethyl-4-piperidincarboxamid und

II. (+)-1-[4-[Ethyl[2-(4-methoxyphenyl)-1-methylethyl]amino]-1-oxobutyl]-N,N-dimethyl-4-piperidincarboxamid und ihre physiologisch verträglichen Salze mit anorganischen oder organischen Säuren noch gänzlich andersgeartete pharmakologische Eigenschaften besitzen, die ihre Anwendung als Mittel zur Behandlung von Erkrankungen des Zentralnervensystems und zur Förderung der cerebralen Durchblutung ermöglichen. Auf Grund ihrer antiemetischen Eigenschaften eignen sich diese Verbindungen auch zur Vorbeugung gegen Reise- und Seekrankheiten und angesichts günstiger Effekte auf die cerebrale Durchblutung zur Anwendung in der Geriatrie und bei Migräne. Vor allem aber zeigen diese Verbindungen infolge ihrer hohen Lipophilie eine gute ZNS-Gängigkeit und lassen sich zur Therapie von Erkrankungen des Zentralnervensystems, insbesondere des Morbus Alzheimer und des Morbus Parkinson, einsetzen.

Die Stimulierung muscarinischer Rezeptoren in endothelgeschädigten Arterien, etwa in Coronar- oder Basilararterien, führt zu einer Konstriktion. Beispielsweise vermindert vagal freigesetztes Acetylcholin den coronaren Blutfluß in isolierten Rattenherzen, Methacholin bewirkt dosisabhängige Kontraktionen der Coronar- und Basilararterien im in-vitro-Experiment (K.J. van Charldorp, Dissertation "Characterisation of Muscarinic Receptors in the Vascular System", Amsterdam 1988; K.J. van Charldorp, D. Davidesko und P.A. van Zwieten, Eur. J. Pharmacol. 150, 197-199 (1988); K.J. van Charldorp und P.A. van Zwieten, Naunyn Schmiedeberg's Arch. Pharmacol. 339, 403-408 (1989)).

Die genannten Untersuchungen geben klare Hinweise darauf, daß die cholinerge Stimulation vasculärer muscarinischer Rezeptoren von pathophysiologischer Bedeutung ist, beispielsweise bei Patienten, die an Prinzmetal-Angina leiden oder cerebralsklerotisch geschädigt sind. Hemmung der in Hirngefäßen nachweisbaren muscarinischen Rezeptoren führt also zu einer Vermeidung der Konstriktionen und zu einer Verbesserung bzw. Normalisierung der arteriosklerotisch bedingten Störungen der Cerebraldurchblutungen. Die Verbindungen der vorliegenden Anmeldung hemmen die in Cerebralgefäßen, beispielsweise in der Pia mater, nachweisbaren muscarinischen Rezeptoren bereits bei physiologisch erreichbaren Konzentrationen bzw. Plasmaspiegeln und sind zur Entwicklung von Therapeutica zur Behandlung arteriosklerotisch bedingter Durchblutungsstörungen des ZNS geeignet.

Bei seniler Demenz vom Alzheimer-Typ führt Degeneration cholinerger Neuronen, insbesondere in hippocampalen und corticalen Projektionen, zu verminderter Freisetzung des Neurotransmitters Acetylcholin. Blockade der präsynaptischen Autorezeptoren unterbricht nun den negativen Rückkopplungsmechanismus, den der Neurotransmitter an den noch intakten Neuronen ausübt, und bewirkt daher einen gesteigerten Acetylcholin-Release und - damit verknüpft - eine Stimulierung der postsynaptischen Rezeptoren (D.C. Mash, D. L. Flynn und L.T. Potter, Science 228, 115-117 (1985); E.K. Perry u. a., Can. J. Neurol. Sci. 13, 521-527 (1986); M. Sarter u. a., TINS 11, 13-17 (1988)).

Beim Morbus Alzheimer beeinflussen die Verbindungen also die autoregulatorische Funktion präsynaptischer Muskarinrezeptoren auf die Freisetzung von Acetylcholin und führen dadurch zu einer Verstärkung des Impulsmusters der noch vorhandenen cholinergischen Fasern; beim Morbus Parkinson besteht der Vorteil des Einsatzes der eingangs erwähnten Verbindungen I und II an Stelle der bisher üblichen nichtselektiven Antimuskarinika in dem Fehlen nicht tolerabler peripherer und zentraler atropinartiger Nebenwirkungen.

Zum Nachweis der günstigen Effekte auf die cerebrale Durchblutung und den vermehrten Acetylcholin-Release wurden die folgenden Versuche durchgeführt:

A. Bindung verschiedener muskarinischer Antagonisten an Rezeptoren der Pia mater des Rindes

Die Pia-Gefäße von Rinder-Hirnen, erhalten vom lokalen Schlachthof, wurden präpariert und von Blutresten gereinigt. Je 0,5 g-Portionen wurden bei 8°C bis zur weiteren Verwendung gelagert. Zur Homogenatgewinnung wurden 0,5 g Pia-Geäße mittels eines Gewebehackers zerkleinert, in 60 ml HEPES-Puffer (20 mM HEPES, 10 mM $MgCl_2$, 100 mM NaCl, pH 7,50) aufgenommen, mit einem Ultra-Turrax homogenisiert und 20 min. bei 0°C mit 48000 x g zentrifugiert. Das Pellet wurde erneut mit einem Ultra-Turrax homogenisiert, durch Mull filtriert und mit HEPES-Puffer auf eine Verdünnung von 1:140 bezogen auf das Feuchtgewicht der Gefäße verdünnt.

0,3 nM [³H]NMS und steigende Konzentrationen des Antagonisten wurden mit dem Homogenat in einem Gesamtvolumen von 0,52 ml für 45 min. bei Raumtemperatur inkubiert. Der Inkubationsansatz wurde mit Hilfe eines Scatron Cell Harvesters über Glasfaser-Filtermatten filtriert und zweimal mit eiskalter physiologischer Kochsalzlösung gewaschen. Die Filter-gebundene Radioaktivität wurde in einem Flüssigkeitsszintillationszähler bestimmt. Als unspezifische Bindung wurde die gebundene Radioaktivität in Gegenwart von $10^{-6}$ M (-)-Chinuclidinyl-benzilat definiert.

B. Transmitter Release Studien

Die Versuche zur in-vitro Freisetzung von Acetylcholin aus hippocampalen Gewebeschnitten wurden in Analogie zu Anordnungen durchgeführt, die für die Freisetzung von 5-HT aus Hypothalamus- bzw. Hippocampus-Gewebe beschrieben sind (vgl. M.H. Richards, Naunyn Schmiedeberg's Arch. Pharmacol. 329, 359-366 (1985) und T.J. Feuerstein, G. Hertting und R. Jackisch, Europ. J. Pharmacol. 107, 233-242 (1985)).

Männliche Ratten (Stamm Chbb:Thom, Gewicht 200 bis 300 g) wurden ohne Vorbehandlung dekapitiert; das gesamte Gehirn wurde sofort entnommen und unter Eiskühlung zerlegt. Gemäß den oben beschriebenen Verfahren wurde der Hippocampus in 0,4 mm dicke Schnitte mit Hilfe eines McIlwain-Gewebehackers geschnitten. Die Gewebeschnitte wurden 30 Minuten lang bei 37°C vorincubiert in Gegenwart von 0,05 mM ³H-Cholin in einem Krebs-Henseleit Puffer der folgenden Zusammensetzung: 120 mM NaCl, 4,8 mM KCl, 1,3 mM $CaCl_2$, 1,2 mM $KH_2PO_4$, 1,2 mM $MgSO_4$ und 25 mM $NaHCO_3$. Der Puffer enthielt zusätzlich 2 g Glucose und 100 mg EDTA. Der Puffer wurde mit Natronlauge auf einen pH-Wert von 7,4 bei 20°C eingestellt. Alle Lösungen wurden kontinuierlich mit 95 % $O_2$ und 5 % $CO_2$ gesättigt. Die Gewebeschnitte wurden daraufhin gespült, jeder Gewebeschnitt wurde jeweils willkürlich auf 12 Superfusionseinheiten verteilt und mit dem Puffer bei 37°C bei einer Fließgeschwindigkeit von 1 ml/min überspült. Die Gewebeschnitte wurden mit einem Polypropylennetzwerk zwischen zwei Platinelektroden gehalten. Nach 50-minütiger Überspülung wurden 4-Minuten-Fraktionen des Superfusats über eine gesamte Zeitdauer von 92 Minuten gesammelt. Die Gewebeschnitte wurden zweimal elektrisch stimuliert, und zwar 60 Minuten (S1) und 100 Minuten (S2) nach Beginn der Superfusion. Dabei wurden Rechteckimpulse von 2 ms Dauer mit einer Frequenz von 3 Hz 100 Sekunden lang bei 5 V/cm und 24 mA mittels eines HSE Stimulators II der Firma Hugo Sachs Elektronik, Hugstetten, West-Deutschland, verabreicht. Die zu prüfenden Substanzen wurden dem Puffer ab 20 Minuten vor dem Zeitpunkt S2 zusammen mit dem Agonisten (Carbachol) beigefügt. Am Ende wurden die Gewebeschnitte aus den Kammern entfernt und zur Bestimmung des verbliebenen Tritiums solubilisiert.

Die Filterscheiben mit der Radioaktivität, die an die Rezeptoren oder an die Membranen gebunden ist, zusammen mit den Synaptosomen wurden in Minivials unter Hinzufügen von 3 ml eines Scintillation Cocktails (Quickszint 2000, Zinsser Analytic, Frankfurt, West-Deutschland) gegeben und eine Stunde lang langsam rotiert. Die Extrakte der MAO-Aktivitätsbestimmung (4 ml) wurden in Standard-Vials mit 10 ml dieses Cocktails übertragen. Die Radioaktivität wurde daraufhin mittels der üblichen Flüssig-Scintillations-Messung bestimmt mit externer Standardisierung in Beckman LS 3800 oder LS 7800 Instrumenten. Die Ergebnisse sind in der Tabelle II enthalten.

C. Bindungsstudien an muscarinischen Rezeptoren des Cortex

Als Organspender dienten männliche Sprague-Dawley-Ratten mit 180-220 g Körpergewicht. Nach Entnahme der Großhirnrinde wurden alle weiteren Schritte in eiskaltem Hepes-HCl-Puffer (pH 7,4; 100 m molar NaCl, 10 m molar $MgCl_2$) vorgenommen. Das Gesamtherz wurde mit einer Schere zerkleinert. Alle Organe wurden abschließend in einem Potter homogenisiert.

Für den Bindungstest wurden die Organhomogenate in folgender Weise verdünnt:

| Gesamtherz | 1: 400 | |
|---|---|---|
| Großhirnrinde | 1:3000 | |
| Submandibularis | 1: 400 | (v:v) |

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30° C. Die Inkubationsdauer betrug 45 Minuten. Als Radioligand wurde 0,3 n molar $^3$H-N-Methylscopolamin ($^3$H-NMS) verwendet. Die Inkubation wurde durch Zugabe von eiskaltem Puffer mit nachfolgender Vakuumfiltration beendet. Die Filter wurden mit kaltem Puffer gespült und ihre Radioaktivität bestimmt. Sie repräsentiert die Summe von spezifischer und unspezifischer Bindung von $^3$H-NMS. Der Anteil der unspezifischen Bindung wurde defi niert als jene Radioaktivität, die in Anwesenheit von 1 $\mu$ molar Chinuclidinylbenzilat gebunden wurde. Es wurden immer Vierfachbestimmungen vorgenommen. Die $IC_{50}$-Werte der nichtmarkierten Testsubstanzen wurden graphisch bestimmt. Sie repräsentieren jene Konzentration der Testsubstanz, bei welcher die spezifische Bindung von $^3$H-NMS an die muscarinischen Rezeptoren in den verschiedenen Organen um 50 % gehemmt wurde. Die Ergebnisse sind aus der Tabelle II zu ersehen.

Die folgenden Tabellen enthalten die dabei gefundenen Ergebnisse:

Tabelle I:

| Hemmung der $^3$H-NMS-Bindung an muscarinische Rezeptoren der Pia mater des Rindes | |
|---|---|
| Substanz | -log $IC_{50}$ (M) |
| I | 8,2 |

Tabelle II:

| Einfluß auf den Transmitter-Release im Vergleich zur Affinität zu Rezeptoren vom $M_1$-Subtyp | | | |
|---|---|---|---|
| Substanz | ACh-Release am Hippocampus-Gewebe ($pA_2$) | Rezeptor-Bingungstests Cortex -log $IC_{50}$ (M) | Verhältnis von $IC_{50}$ (Cortex) zu $pA_2$(ACh-Release) |
| I | 8,7 | 7,3 | 25 |

Die vorstehende Tabelle I zeigt, daß die Verbindungen der vorliegenden Anmeldung muscarinische Rezeptoren der Cerebralgefäße - als Beispiel wurden solche der Pia mater gewählt - bereits bei physiologisch erreichbaren Konzentrationen bzw. Plasmaspiegeln zu hemmen vermögen.

Die vorstehende Tabelle II beweist, daß die Verbindungen der vorliegenden Anmeldung nicht nur bereits bei sehr niedrigen Konzentrationen die inhibitorischen Autorezeptoren in hippocampalem Gewebe blockieren und damit die Acetylcholin-Freisetzung erhöhen sondern darüber hinaus auch selektiv sind, da sie erst bei merklich höheren Konzentrationen postsynaptische Rezeptoren vom $M_1$-Subtyp hemmen. Die Verbindungen ermöglichen also einen Ausgleich der für senile Demenz vom Alzheimer-Typ charakteristischen cholinergen Defizite und eignen sich zur Verwendung als Arzneimittel zur Bekämpfung des Morbus Alzheimer.

Gegenstand der Erfindung ist die Verwendung von Arzneimitteln als Antiemetika, als Mittel zur Förderung der cerebralen Durchblutung, als Antimigränemittel, insbesondere aber zur Bekämpfung des Morbus Alzheimer und Morbus Parkinson, die die Verbindung (±)-1-[4-[Ethyl[2-(4-methoxyphenyl)-1-methylethyl]amino]-1-oxobutyl]-N,N-dimethyl-4-piperidincarboxamid (I) und (+)-1-[4-[Ethyl[2-(4-methoxyphenyl)-1-methyl- ethyl]amino]-1-oxobutyl]-N,N-dimethyl-4-piperidincarboxamid (II) sowie deren physiologisch verträgliche Salze mit anorganischen oder organischen Säuren enthalten.

Die Verbindungen I und II und deren physiologisch verträgliche Salze lassen sich hierzu in an sich bekannter Weise in die üblichen pharmazeutischen Zubereitungsformen, z.B. in Lösungen, Suppositorien, Tabletten, Dragées, Kapseln oder Teezubereitungen einarbeiten. Die Tagesdosis liegt im allgemeinen zwischen 0,02 und 5 mg/kg, vorzugsweise 0,02 und 2,5 mg/kg, insbesondere 0,05 und 1,0 mg/kg Körpergewicht, die gegebenenfalls in Form mehrerer, vorzugsweise 1 bis 3 Einzelgaben, zur Erzielung der gewünschten Ergebnisse verabreicht wird.

Die Herstellung der Verbindungen I und II wird in der europäischen Patentanmeldung Nr. 85 201 394.5 (Veröffentlichungs-Nr. 0 177 078) beschrieben.

Im folgenden wir die Herstellung pharmazeutischer Anwendungsformen anhand einiger Beispiele beschrieben.:

Beispiel I

Tabletten mit 20,0 mg (±)-1-[4-[Ethyl[2-(4-methoxyphenyl)1-methylethyl]amino]-1-oxobutyl]-N,N-dimethyl-4-piperidin-carboxamid

| Zusammensetzung: | |
|---|---|
| 1 Tablette enthält: | |
| Wirkstoff | 20,0 mg |
| Milchzucker | 152,0 mg |
| Kartoffelstärke | 65,0 mg |
| Magnesiumstearat | 2,0 mg |
| | 239,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45° C getrocknet, nochmals durch obiges Sieb gerieben, mit Magnesiumstearat vermischt und zu Tabletten verpreßt.
Tablettengewicht: 239 mg
Stempel: 9 mm

Beispiel II

Dragées mit 20,0 mg (±)-1-[4-[Ethyl[2-(4-methoxyphenyl)1-methylethyl]amino]-1-oxobutyl]-N,N-dimethyl-4-piperidin-carboxamid

Die nach Beispiel I hergestellten Tabletten werden nach bekanntem Verfahrem mit einer Hülle überzogen, die im wesentlichen aus Zucker und Talkum besteht. Die fertigen Dragées werden mit Hilfe von Bienenwachs poliert.
Drageegewicht: 300 mg

Beispiel III

Ampullen mit 4,0 mg (±)-1-[4-[Ethyl[2-(4-methoxyphenyl)1-methylethyl]amino]-1-oxobutyl]-N,N-dimethyl-4-piperidin-carboxamid

| Zusammensetzung: | | |
|---|---|---|
| 1 Ampulle enthält: | | |
| Wirkstoff | | 4,0 mg |
| Natriumchlorid | | 8,0 mg |
| Dest. Wasser | ad | 1 ml |

Herstellungsverfahren:

Die Wirksubstanz und Natriumchlorid werden in dest. Wasser gelöst und anschließend auf das gegebene Volumen aufgefüllt. Die Lösung wird sterilfiltriert und in 1ml-Ampullen abgefüllt.
Sterilisation: 20 Minuten bei 120° C.

Beispiel IV

Suppositorien mit 20,0 mg (±)-1-[4-[Ethyl[2-(4-methoxyphenyl)1-methylethyl]amino]-1-oxobutyl]-N,N-dimethyl-4-piperidin-carboxamid

| Zusammensetzung: | |
|---|---|
| 1 Zäpfchen enthält: | |
| Wirkstoff | 20,0 mg |
| Zäpfchenmasse (z.B. Witepsol W 45 [R]) | 1690,0 mg |
| | 1710,0 mg |

Herstellungsverfahren:

Die feinpulverisierte Wirksubstanz wird in der geschmolzenen und auf 40° C abgekühlten Zäpfchenmasse suspendiert. Man gießt die Masse bei 37° C in leicht vorgekühlte Zäpfchenformen aus. Zäpfchengewicht: 1,71 g

Beispiel V

Tropfen mit (±)-1-[4-[Ethyl[2-(4-methoxyphenyl)1-methylethyl]amino]-1-oxobutyl]-N,N-dimethyl-4-piperidin-carboxamid

| Zusammensetzung: | | |
|---|---|---|
| 100 ml Tropflösung enthalten: | | |
| p-Hydroxybenzoesäuremethylester | | 0,035 g |
| p-Hydroxybenzoesäurepropylester | | 0,015 g |
| Anisöl | | 0,05 g |
| Menthol | | 0,06 g |
| Ethanol rein | | 10,0 g |
| Wirkstoff | | 0,8 g |
| Natriumcyclamat | | 1,0 g |
| Glycerin | | 15,0 g |
| Dest. Wasser | .. ad | 100,0 ml |

Herstellungsverfahren:

Die Wirksubstanz und Natriumcyclamat werden in ca. 70 ml Wasser gelöst und Glycerin zugefügt. Man löst die p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert. .

**Ansprüche**

1.) Verwendung von (±)-1-[4-[Ethyl[2-(4-methoxyphenyl)-1-methylethyl]amino]-1-oxobutyl]-N,N-dimethyl-4-piperidincarboxamid und ( + )-1-[4-[Ethyl[2-(4-methoxyphenyl)-1-methylethyl]amino]-1-oxobutyl]-N,N-dimethyl-4-piperidincarboxamid bzw. von physiologisch verträglichen Salzen dieser Verbindungen mit anorganischen oder organischen Säuren zur Behandlung von Erkrankungen des Zentralnervensystems.

2.) Verwendung von (±)-1-[4-[Ethyl[2-(4-methoxyphenyl)-1-methylethyl]amino]-1-oxobutyl]-N,N-dimethyl-4-piperidincarboxamid und ( + )-1-[4-[Ethyl[2-(4-methoxyphenyl)-1-methylethyl]amino]-1-oxobutyl]-N,N-dimethyl-4-piperidincarboxamid bzw. von physiologisch verträglichen Salzen dieser Verbindungen mit anorganischen oder organischen Säuren zur Herstellung eines Arzneimittels zur Behandlung von Erkrankungen des Zentralnervensystems.

3.) Verwendung von Verbindungen des Anspruchs 1 und 2 zur Bekämpfung des Morbus Alzheimer.

4.) Verwendung von Verbindungen des Anspruchs 1 und 2 zur Bekämpfung des Morbus Parkinson.

5.) Verwendung der im Anspruch 1 und 2 genannten Verbindungen zur Herstellung von Antiemetika.

6.) Verwendung der im Anspruch 1 und 2 genannten Verbindungen zur Herstellung von Mitteln zur Förderung der cerebralen Durch- blutung.

7.) Verwendung der in Anspruch 1 und 2 genannten Verbindungen zur Herstellung von Mitteln gegen Migräne.